# EUROPÄISCHE PATENTANMELDUNG

(11) **EP 0 781 759 A2**
(43) Veröffentlichungstag der Anmeldung: **02.07.1997**
(21) Anmeldenummer: 96120777.6
(22) Anmeldetag: 23.12.1996
(51) Int. Cl.: C07C 67/08, C07C 69/54

(54) **Verfahren zum Verestern von (Meth)acrylsäure mit einem Alkanol**

(30) Priorität: 27.12.1995 DE 19548912
(71) Anmelder: BASF AKTIENGESELLSCHAFT, 67056 Ludwigshafen (DE)
(72) Erfinder: Annen, Ulrich, Dr., 67454 Hassloch (DE); Baur, Karl Gerhard, Dr., 67063 Ludwigshafen (DE); Dockner, Toni, Dr., 67149 Meckenheim (DE)
(74) Vertreter: Isenbruck, Günter, Dr.

(57) **Zusammenfassung**

Die Erfindung betrifft ein Verfahren zum Verestern von (Meth)acrylsäure mit einem Alkanol in Gegenwart eines sauren Veresterungskatalysators, bei dem nicht umgesetzte Ausgangsverbindungen und der gebildete (Meth)acrylsäureester destillativ abgetrennt werden und ein Oxyester enthaltendes Sumpfprodukt entsteht, das abgetrennt und verdampft wird, und der Dampf bei erhöhter Temperatur in Gegenwart eines Katalysators gespalten wird, wobei ein basischer Katalysator eingesetzt wird.

## Beschreibung

Die Erfindung betrifft ein Verfahren zum Verestern von (Meth)acrylsäure mit einem Alkanol in Gegenwart eines Veresterungskatalysators, bei dem nicht umgesetzte Ausgangsverbindungen und der gebildete (Meth)acrylsäureester destillativ abgetrennt werden und ein Oxyester enthaltendes Sumpfprodukt entsteht. Der Begriff (Meth)acrylsäure bezeichnet dabei in bekannter Weise Acryl- oder Methacrylsäure.

Die Herstellung von Alkylestern der (Meth)acrylsäure erfolgt üblicherweise durch Verestern von (Meth)acrylsäure mit Alkanolen bei erhöhter Temperatur in flüssiger Phase mit oder ohne Lösungsmittel und in Gegenwart von Säure als Katalysator (DE-A 23 39 519). Nachteilig an dieser Herstellungsweise ist, daß sich unter den vorgenannten Veresterungsbedingungen als Nebenreaktionen noch nicht umgesetzter Ausgangsalkohol unter Ausbildung einer Verbindung der nachfolgend angeführten allgemeinen Formel I sowie noch nicht umgesetzte (Meth)acrylsäure unter Ausbildung einer Verbindung der allgemeinen Formel II an die Doppelbindung von bereits gebildetem (Meth)acrylsäurealkylester addiert (Michael-Addition). Auch ein Mehrfachaddition ist möglich. Ferner können gemischte Typen auftreten. Diese Addukte (Alkoxyester und Acyloxyester) nennt man kurz Oxyester. 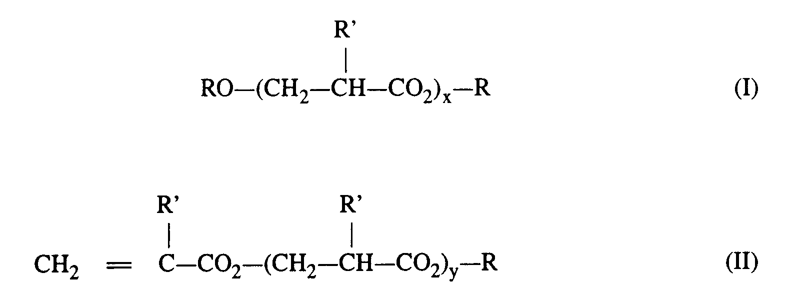 mit
x,y = 1-5
R = Alkyl
R' = H oder CH₃

Wenn R' = H ist, handelt es sich um eine Veresterung der Acrylsäure, wenn R' = CH₃ ist, handelt es sich um eine Veresterung der Methacrylsäure.

Bei der Herstellung von Estern der Acrylsäure ist das Problem der Oxyesterbildung besonders akut, wobei die hauptsächlich gebildeten Oxyester der Alkoxypropionsäureester und der Acyloxypropionsäureester mit x,y = 1 sind. Bei der Herstellung von Estern der Methacrylsäure erfolgt die Oxyesterbildung in geringerem Maße. Die Entstehung von Oxyestern ist in der DE-A 23 39 529 beschrieben. Aus dieser geht hervor, daß die Bildung von Oxyestern im wesentlichen unabhängig von den speziellen Veresterungsbindungen erfolgt. Von ganz besonderer Bedeutung ist die Oxyesterbildung bei der Herstellung von Acrylaten der C₁- bis C₈-Alkanole, insbesondere der C₄- bis C₈-Alkanole, ganz besonders bei der Herstellung von n-Butylacrylat und 2-Ethylhexylacrylat.

Charakteristisch für die Oxyester ist, daß ihr Siedepunkt oberhalb der Siedepunkte von Ausgangssäure, Ausgangsalkohol, gebildetem Zielester sowie gegebenenfalls mitverwendetem organischem Lösungsmittel liegt.

Die Aufarbeitung eines beliebigen Veresterungs-Reaktionsgemisches erfolgt normalerweise so, daß nicht umgesetzte Ausgangsverbindungen sowie der Zielester vom Reaktionsgemisch destillativ abgetrennt werden, wobei der zur Veresterung verwendete Säurekatalysator gegebenenfalls vorher durch Extraktion mittels Wasser und/oder wäßriger Lauge abgetrennt wird (vgl. z.B. Ullmann's Encyclopedia of Industrial Chemistry, Vol. A1, 5th Ed., VCH, S. 167ff). Das im Rahmen einer solchen destillativen Aufarbeitung verbleibende Sumpfprodukt enthält die Oxyester, die einen beträchtlichen Ausbeuteverlust bedingen.

Es sind daher weitere verschiedenartige Verfahren untersucht worden, um die durch das Auftreten der Oxyester entstehenden Probleme zu lösen. So ist aus der DE-A-1 124 482 bekannt, die Oxyester in der Gasphase in Gegenwart sauerwirkender Katalysatoren auf Trägern zu spalten, wobei saure Erdalkaliphosphate auf Kieselsäuregel als besonders vorteilhaft genannt sind. Weiterhin ist aus der DE-A-1 124 483 ein ähnliches Verfahren, bei dem als Katalysator Aluminiumoxid und geringe Mengen von Oxiden der VI. Nebengruppe des Periodensystems verwendet wurden. Ferner ist aus der DE-A-1 126 378 ein Verfahren bekannt, in dem Borphosphat als Katalysator verwendet wurde. Schließlich ist aus der EP-A2-29 800 bekannt, kristalline Aluminiumsilikate in Form von Zeolithen einzusetzen, die mit einem Alkalimetall aus der Gruppe 1A des Periodensystems und einem Metall aus der Gruppe VIIIB des Periodensystems modifiziert worden sind. Es hat sich jedoch gezeigt, daß bei Verwendung von sauren Katalysatoren unerwünschte Nebenprodukte, wie Dialkylether, entstehen. Die Verwendung von Zeolithen führt ebenfalls zur Bildung von Dialkylether als Nebenprodukt.

Der Erfindung liegt die Aufgabe zugrunde, im Rahmen der Veresterung von (Meth)acrylsäure mit einem Alkanol und der destillativen Abtrennung des Zielesters die im Sumpfprodukt enthaltenen Oxyester rückzuspalten und die dabei anfallenden Ausgangssäure, Ausgangsalkohol und Zielester ohne die Nachteile der Verfahren nach dem Stand der Technik einer Weiterverwendung zuzuführen.

Dies wird erfindungsgemäß erreicht durch ein Verfahren zum Verestern von (Meth)acrylsäure mit einem Alkanol in Gegenwart eines sauren Veresterungskatalysators, bei dem nicht umgesetzte Ausgangsverbindungen und der gebildete (Meth)acrylsäureester destillativ abgetrennt werden und ein Oxyester enthaltendes Sumpfprodukt entsteht, das abgetrennt und verdampft wird, wobei der Dampf bei erhöhter Temperatur in Gegenwart eines Katalysators gespalten wird, dadurch gekennzeichnet, daß ein basischer Katalysator eingesetzt wird. Dieser basische Katalysator besteht vorzugsweise aus einer oder mehreren Oxiden von Alkalimetallen der Gruppe IIa des Periodensystems, insbesondere kommen hierfür in Frage Oxide der Alkali- und/oder Erdalkalimetalle. Der Katalysator ist vorzugsweise ein Festbettkatalysator, der im Reaktor angeordnet ist. Der Katalysator kann sich auf einem Trägermaterial befinden. Dies ist insbesondere bei Oxiden der Alkalielemente notwendig. Bei Oxiden der Erdalkalimetalle, insbesondere bei Magnesiumoxid, ist kein Trägermaterial erforderlich. Als Trägermaterial kann jedes bekannte Trägermaterial dienen. Bevorzugt ist hierbei Titandioxid, jedoch sind auch andere, vorzugsweise poröse Trägermaterialien, wie Kieselsäuregel und Zeolithe, einsetzbar.

Die Arbeitstemperatur im Reaktor bei der Spaltung liegt in der Regel bei 200°C bis 400°C, vorzugsweie bei 250°C bis 350°C. Vorzugsweise herrscht im Reaktor Unterdruck, um die zu spaltenden Oxyester sicher in der Gasphase zu halten und keine Kondensation zu ermöglichen. Der Arbeitsdruck liegt dabei zwischen 10 mbar und 500 mbar, vorzugsweise bei 100 mbar bis 250 mbar. Vorteilhafterweise wird dem zu spaltenden Dampf im Reaktor ein Treibgas zugesetzt, um die Verweilzeit im Reaktor gering zu halten. Hierfür empfiehlt sich ein inertes Gas, wie Stickstoff. Es kann jedoch auch Luft verwendet werden.

Der Umsatz im Reaktor liegt zwischen 15 und 55 Gew.-% pro Durchgang des zugeführten Dampfes. Die Verweilzeit (VwZ) im Reaktor beträgt 0,1 bis 1 sec, vorzugsweise 0,15 bis 0,25 sec. Diese Verweilzeit muß möglichst gering gehalten werden, um eine Weiterspaltung zu vermeiden. Sie muß außerdem gering sein, weil sonst auch Polymersationen der gebildeten Spaltprodukte auftreten können. Die Katalysatorbelastung kann 0,02 bis 0,2 g/gh, vorzugsweise 0,05 bis 0,15 g/gh betragen.

Beim erfindungsgemäßen Verfahren wird das bei der Veresterung von (Meth)acrylsäure mit einem Alkanol entstandene Sumpfprodukt, das die Oxyester enthält, zunächst destillativ in Oxyester und höhersiedende Bestandteile zerlegt. Die Oxyester werden dann gasförmig abgezogen, auf Arbeitstemperatur erhitzt und danach über den Katalysator geführt. Das durch die katalytische Spaltung entstehende Produktgemisch wird vorteilhafterweise durch partielle Kondensation aufgetrennt. Zunächst kondensiert man die nicht umgesetzten Oxyester aus und führt sie erneut der Spaltung zu. Die bei dieser Teilkondensation gasförmig verbleibenden leichtsiedenden Spaltprodukte können nach einer weiteren Kondensation in die Veresterung zurückgeführt werden. Durch diese Verfahrensweise wird eine hohe Ausbeute des Gesamtverfahrens erreicht und die Bildung von unerwünschten Nebenprodukten vermieden.

Von besonderem Vorteil ist das Verfahren bei der Veresterung von Alkanolen mit mehr als zwei C-Atomen vorzugsweise mit zwei bis vier C-Atomen.

Weitere Einzelheiten und Vorteile der Erfindung können den anhand des Verfahrensschemas erläuterten Ausführungsbeispielen entnommen werden.

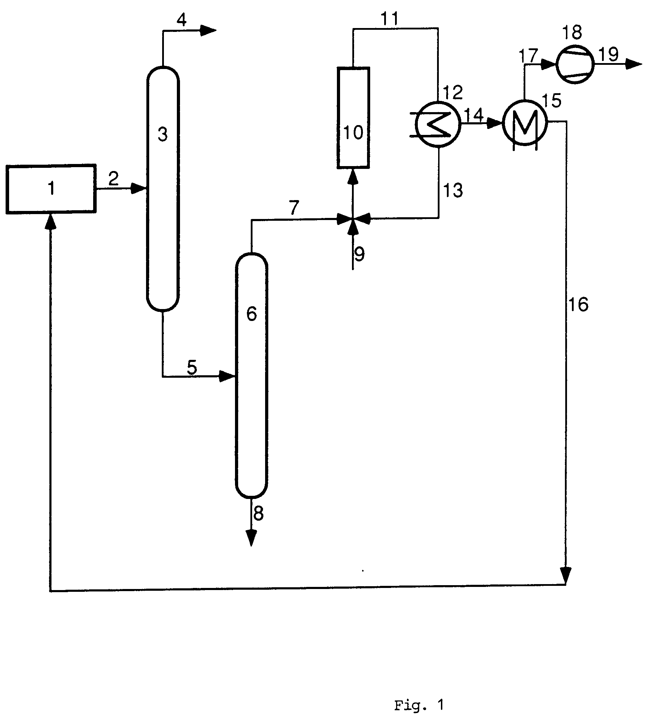

Fig. 1 zeigt dabei das Verfahrensschema.

Dabei wird der zu bildende (Meth)acrylsäureester nach bekannten Verfahren hergestellt. Die entsprechenden Apparaturen sind schematisch durch den

Block 1 dargestellt. Über die Leitung 2 wird der gebildete (Meth)acrylsäureester zusammen mit den dabei ebenfalls gebildeten Oxyestern und Hochsieder einer Rektifikationskolonne 3 zugeführt. In dieser Kolonne wird das zugeführte Gemisch aufgetrennt in den zu bildenden (Methy)acrylsäureester, der durch die Leitung 4 abgezogen werden kann, und ein Sumpfprodukt, das vorzugsweise die Oxyester und Hochsieder enthält und das durch die Leitung 5 einer weiteren Kolonne 6 zugeleitet wird. Diese dient der Trennung der Oxyester von den Hochsiedern. Die Oxyester werden vom Kopf dieser Kolonne 6 über die Leitung 7 abgezogen, während die Hochsieder über die Leitung 8 vom Sumpf der Kolonne 6 abgezogen werden.

Die dampfförmigen Oxyester werden über die Leitung 7 in den Spaltreaktor 10 eingeführt, dem über eine Leitung 9 auch Stickstoff zugeführt wird. Der Spaltrektor 10 ist ein Festbettreaktor, der den erfindungsgemäßen Katalysator enthält. Die im Reaktor 10 entstehenden Spaltprodukte, die aus Alkanol, (Meth)acrylsäureester, verbliebenen Oxyestern und Stickstoff bestehen, werden durch die Leitung 11 abgezogen und einem Kondensator 12 zugeführt, in dem eine partielle Kondensation der verbliebenen Oxyester stattfindet, die dann durch Leitung 13 erneut dem Reaktor 10 zugeführt werden.

Das im Kondensator 12 gasförmig gebliebene Gemisch aus Alkanol, (Meth)acrylsäureester und Stickstoff wird durch die Leitung 14 einem weiteren Kondensator 15 zugeführt, in dem Alkanol und (Meth)acrylsäureester kondensiert und über die Leitung 16 dem Veresterungsverfahren 1 wieder zugeführt werden. Der Kondensator 15 ist über eine Leitung 17 mit einer Vakuumanlage 18 verbunden, aus der durch Leitung 19 den zugeführten Stickstoff enthaltendes Abgas abgeführt wird.

In einer so aufgebauten Anlage wurden versuchsweise die bei der Synthese von n-Butylacrylat aus n-Butanol und Acrylsäure in Gegenwart eines Katalysators, wie Paratoluolsulfonsäure entstandenen Gemische, die neben dem Zielester, nämlich n-Butylacrylat, die unerwünschten Oxyester enthielten, aufgearbeitet.

Als Oxyester trat hier insbesondere Butoxypropionsäurebutylester und Acryloxypropionsäurebutylester auf. Das aus dem Veresterungsprozeß stammende Butylacrylat/Hochsieder-Gemisch wurde mit der Leitung 2 in die Kolonne 3 eingeführt, dort aufgetrennt in das am Kopf durch die Leitung 4 abgezogene n-Butylacrylat und in ein Sumpfprodukt, das die Oxyester enthielt und über Leitung 5 der Kolonne 6 zugeführt wurde. Das vom Kopf dieser Kolonne 6 über die Leitung 7 abströmende Gemisch gasförmiger Oxyester wurde zusammen mit über die Leitung 9 zuströmendem Stickstoff dem Spaltreaktor 10 zugeführt. In diesem wurde die Spaltung versuchsweise mit verschiedenen Katalysatoren durchgeführt. Bei einer Versuchsreihe wurde als Katalysator Magnesiumoxid (MgO) eingesetzt. Eine weitere Versuchsreihe wurde mit Kaliumoxid (K₂O) als Katalysator durchgeführt.

### 1. Katalysator MgO

Die Versuchsbedingungen bei Verwendung von MgO als Katalysator sind in der folgenden Tabelle 1 niedergelegt. Dabei wurde MgO ohne Trägermaterial eingesetzt. Verwendet wurden die unter der Bezeichnung MgO S9-85 von der Fa. BASF vertriebenen Katalysatoren. Hierbei handelte es sich um Vollkontakt-Tabletten 5 x 3 mm. Das Rüttelgewicht betrug 1100 g/l, die Porösität 0,35 ml/g, die Oberfläche 65 m²/g.

Der Druck im Reaktor betrug bei diesen Versuchen 100 mbar. Die Menge an zugeführtem Stickstoff betrug 20 l/h.

**Tabelle 1**

| Katalysator MgO | | | | | |
|---|---|---|---|---|---|
| Temp. (°C) | Durchsatz (g/h) | N₂ (l/h) | Katbelast. (g/gh) | Umsatz (%) | VwZ (s) |
| 250 | 10 | 20 | 0,06 | 19 | 0,16 |
| 280 | 10 | 20 | 0,06 | 26 | 0,15 |
| 330 | 10 | 20 | 0,06 | 46 | 0,14 |
| 330 | 24 | 20 | 0,13 | 35 | 0,14 |
| 350 | 24 | 20 | 0,13 | 40 | 0,13 |
| 380 | 24 | 20 | 0,13 | 46 | 0,12 |

Bei allen angeführten Versuchen konnte kein Nebenprodukt, also kein Di-n-Butylether in dem durch die Leitung 14 aus dem Kondensator 12 abgezogenen Gemisch mehr festgestellt werden. Dies gilt demgemäß auch für das aus dem weiteren Kondensator 15 durch die Leitung 16 dem Veresterungsprozeß zugeführten, aus Butanol und n-Butylacrylat bestehenden Gemisch.

### 2. Katalysator K₂O

Entsprechende Versuche ebenfalls mit den bei der Veresterung von n-Butanol mit Acrylsäure entstehenden Oxyestern wurde mit Kaliumoxid (K₂O) auf Titandioxid als Trägermaterial durchgeführt. Derartige Katalysatoren werden unter der Bezeichnung S9 - 90 von der Fa. BASF vertrieben.

Sie enthalten 1,5 Gew.-% K₂O, ihr Rüttelgewicht beträgt 1000 g/l, ihre Porösität beträgt 93 ml/g, ihre Oberfläche 45 m²/g. Es wurden Stränge mit einem Durchmesser von 4 mm eingesetzt.

Die bei Verwendung dieses Katalysators angewandten Versuchsbedingungen sind der folgenden Tabelle 2 zu entnehmen. Der Druck im Reaktor betrug auch hier 100 mbar. Der durch Leitung 9 zugeführte Stickstoffstrom betrug 20 l/h.

**Tabelle 2**

| **Katalysator TiO**_{**2**}**/K**_{**2**}**O** | | | | |
|---|---|---|---|---|
| Temp.(°C) | Durchsatz (g/h) | Kat.belastung (g/gh) | Umsatz (%) | VwZ (s) |
| 250 | 6 | 0,03 | 32 | 0,16 |
| 250 | 12 | 0,06 | 28 | 0,16 |
| 250 | 24 | 0,13 | 26 | 0,16 |
| 280 | 12 | 0,06 | 52 | 0,15 |

Auch hier konnte festgestellt werden, daß bei den angegebenen Versuchsbedingungen keinerlei Nebenprodukte entstanden sind.

## Patentansprüche

1. Verfahren zum Verestern von (Meth)acrylsäure mit einem Alkanol in Gegenwart eines sauren Veresterungskatalysators, bei dem nicht umgesetzte Ausgangsverbindungen und der gebildete (Meth)acrylsäureester destillativ abgetrennt werden und ein Oxyester enthaltendes Sumpfprodukt entsteht, das abgetrennt und verdampft wird, wobei der Dampf bei erhöhter Temperatur in Gegenwart eines Katalysators gespalten wird, dadurch gekennzeichnet, daß ein basischer Katalysator eingesetzt wird.

2. Verfahren nach Anspruch 1, dadurch gekennzeichnet, daß als basischer Katalysator einer oder mehrere Oxide von Alkalimetallen oder Metallen der Gruppe IIA des Periodensystems verwendet werden.

3. Verfahren nach Anspruch 2, dadurch gekennzeichnet, daß als basischer Katalysator Oxide von Alkali- und/oder Erdalkalimetallen verwendet werden.

4. Verfahren nach Anspruch 1, 2 oder 3, dadurch gekennzeichnet, daß sich der basische Katalysator auf einem Trägermaterial befindet.

5. Verfahren nach Anspruch 4, dadurch gekennzeichnet, daß als Trägermaterial Titandioxid verwendet wird.

6. Verfahren nach einem der vorhergehenden Ansprüche, dadurch gekennzeichnet, daß die Arbeitstemperatur bei der Spaltung 200 °C bis 400 °C, vorzugsweise 250 °C bis 350 °C, beträgt und/oder der Arbeitsdruck bei der Spaltung 10 bis 500 mbar, vorzugsweise 100 bis 250 mbar, beträgt und/oder die Verweilzeit im Reaktor bei der Spaltung 0,1 bis 1 sec, vorzugsweise 0,15 bis 0,25 sec beträgt und/oder die Katalysatorbelastung bei der Spaltung 0,02 bis 0,2 g/gh, vorzugsweise 0,05 bis 0,15 g/gh beträgt.

7. Verfahren nach einem der vorhergehenden Ansprüche, dadurch gekennzeichnet, daß dem zu spaltenden Dampf ein vorzugsweise inertes Treibgas, insbesondere Stickstoff, zugesetzt wird.

8. Verfahren nach einem der vorhergehenden Ansprüche, dadurch gekennzeichnet, daß das zu spaltende Sumpfprodukt zunächst destillativ in Oxyester und höhersiedende Bestandteile zerlegt wird.

9. Verfahren nach Anspruch 8, dadurch gekennzeichnet, daß die Oxyester gasförmig abgezogen, auf Arbeitstemperatur erhitzt und danach über den Katalysator geführt werden.

10. Verfahren nach einem der vorhergehenden Ansprüche, dadurch gekennzeichnet, daß das durch die katalytische Spaltung entstehende Produktgemisch gekühlt, verbliebene Oxyester dabei kondensiert und der Spaltung erneut zugeführt und die leichtsiedenden Produkte dem Veresterungsverfahren wieder zugeführt werden.
